# EUROPEAN PATENT APPLICATION

(11) **EP 2 660 252 A2**
(43) Date of publication of application: **06.11.2013**
(21) Application number: 11853816.4
(22) Date of filing: 29.12.2011
(51) Int. Cl.: C08B 37/00, C08G 65/325, A61K 8/73, A61Q 19/00, A61Q 5/00

(54) **POLYSACCHARIDE-BASED GRAFT COPOLYMER AND COMPOSITION COMPRISING SAME FOR PERSONAL CARE**

(30) Priority: 30.12.2010 KR 20100139638; 25.02.2011 KR 20110017152
(71) Applicant: Kci Limited, Seoul 153-768 (KR)
(72) Inventor: LEE, Mae In, Siheung-si Gyeonggi-do 429-778 (KR); HAN, In Sun, Seoul 156-030 (KR); LEE, Ho Jin, Seoul 138-170 (KR); KIM, Han Soo, Seoul 153-030 (KR)
(74) Representative: Capasso, Olga
(86) International application number: PCT/KR2011/010331
(87) International publication number: WO 2012/091502

(57) **Abstract**

The present invention relates to a graft copolymer including a polysaccharide as a main chain and a phosphorylcholine analogous group-containing monomer as a side chain, and a composition for skin and hair care including the copolymer as an active ingredient. The graft copolymer according to the present invention exhibits more improved moisturizing capacity and better biocompatibility than the existing polysaccharide-based graft copolymer by introducing a phosphorylcholine analogous group-containing compound, and accordingly, the composition for hair and skin care including the same also has improved moisturizing capacity, protection capacity of damaged hair, skin affinity, a skin barrier function, and the like.

## Description

### [Technical Field]

The present invention relates to a new graft copolymer and a composition using the same for personal care, and more particularly, to a graft copolymer which includes polysaccharides as a main chain and other monomers as a side chain. Further, the present invention relates to a composition for personal care, which includes the copolymer as an active ingredient.

### [Background Art]

A graft copolymer of polysaccharide including cellulose in the related art has been developed and used as an active ingredient of a composition for hair care, or for improving dyeability of textile. As a monomer introduced into a polysaccharide substrate in the copolymer, dimethyldiallyl ammonium halide has been used in most cases.

For example, US Patent No. 4,803,071 discloses a composition for hair care which includes a graft copolymer of hydroxyethyl cellulose (hereinafter abbreviated as HEC) and dimethyldiallyl ammonium chloride as an active ingredient.

However, as damage to hair and scalp due to environmental pollution, exposure to UV rays, frequent permanents or dyeing, and the like has been severe, there is an increasing need for new hair and skin products which may exhibit better moisturizing capacity during and after application to hair, provide more improved protecting effects to damaged hair and scalp, and supply environmental friendliness and appropriate nutrition along with the above-described characteristics.

Meanwhile, a phosphorylcholine analogous group-containing polymer results from a structure similar to phospholipids, which is derived from a biomembrane, and it is known that the polymer has excellent properties in terms of deactivation of blood ingredients, biocompatibility such as non-absorptivity to biomaterials and the like, antifouling property, moisture retention, and the like. Moreover, research and development have been actively conducted on the synthesis of a phosphorylcholine analogous group-containing polymer and the use thereof for the purpose of developing organism-related materials which retain the functions.

Examples of the phosphorylcholine analogous group-containing polymer include phosphorylcholine analogous group-containing monomers, and it is known that among them, particularly a monomer referred to as 2-(methacryloyloxy)ethyl-2'-(trimethylammonio)ethylphosphate (also referred to as 2-methacryloyloxyethylphosphorylcholine, and hereinafter, abbreviated as MPC) has a phosphorylcholine analogous group which is structurally similar to phosphatidyl choline that is a constitutional ingredient of a cell wall and thus imparts excellent biocompatibility and superior moisture retention.

### [Detailed Description of the Invention]

### [Technical Problem]

The present invention has been made in an effort to improve the moisturizing capacity and protection capacity of a composition for hair care in the related art, and an object of the present invention is to provide a new polysaccharide-based graft copolymer that provides improved moisturizing capacity and protection capacity and a composition for hair and skin care including the same.

### [Technical Solution]

The object of the present invention may be achieved by providing a graft copolymer including at least one of phosphorylcholine analogous group-containing first monomers represented by the following Formula (1) in at least one of polysaccharides as a main chain.

In addition, the object of the present invention may be achieved by a graft copolymer further including a second monomer including at least one of quaternary ammonium compounds containing an epoxy group.

In the present invention, the contents of the polysaccharide and the phosphorylcholine analogous group-containing monomer, which are included in the graft copolymer, are preferably from 30% by weight to 99.5% by weight and from 0.5% by weight to 70% by weight, respectively.

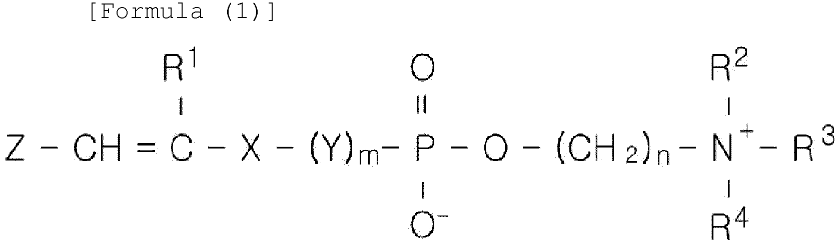

In Formula (1), X represents a divalent organic residue, Y represents an alkyleneoxy group having from 1 to 6 carbon atoms, and Z represents a hydrogen atom or R⁵-O-CO- (here, R⁵ represents an alkyl group having from 1 to 10 carbon atoms, or a hydroxyalkyl group having from 1 to 10 carbon atoms). Furthermore, R¹ represents a hydrogen atom or a methyl group, R², R³, and R⁴ are the same or different groups and represent a hydrogen atom and a hydrocarbon group or a hydroxyhydrocarbon group having from 1 to 6 carbon atoms. m is 0 or 1, and n is an integer from 2 to 4.

Further, in the graft copolymer of the present invention, the type of polysaccharide is not particularly limited, but hydroxyethyl cellulose is preferred from the viewpoint of moisture retention and biocompatibility, and even the types of first monomer and second monomer are not particularly limited, but the first monomer is preferably 2-(methacryloyloxy)ethyl-2'(trimethylammonio)ethylphosphate from the viewpoint of moisture retention and biocompatibility. In addition, the graft copolymer of the present invention may additionally include other monomers in addition to the phosphorylcholine analogous group-containing monomer as a constitutional ingredient, and may additionally include, for example, at least one of dially dialkyl ammonium salts or epoxypropyl trimethyl ammonium chlorides. Furthermore, the weight average molecular weight of the graft copolymer of the present invention is not particularly limited, but is preferably from 10,000 to 2,000,000 from the viewpoint of solubility and moisture retention.

Another object of the present invention may be achieved by providing a composition for hair and skin care including the aforementioned graft copolymer as an active ingredient.

In the composition for hair and skin care of the present invention, the content of the graft copolymer is not particularly limited, but is preferably from 0.01% by weight to 5% by weight from the viewpoint of moisture retention and protection.

### [Advantageous Effects]

The graft copolymer according to the present invention exhibits more improved moisturizing capacity and better biocompatibility than the existing polysaccharide-containing graft copolymer by introducing a phosphorylcholine analogous group-containing monomer into a polysaccharide, and accordingly, the composition for hair and skin care including the same also has improved moisturizing capacity, protection capacity for damaged skin and hair, and the like.

### [Best Mode]

The present invention relates to a new polysaccharide-based graft copolymer which is available for hair and skin products. The graft copolymer of the present invention is a graft copolymer in which a first monomer and/or a second monomer are or is bonded as a side chain to at least one of polysaccharides as a main chain, and the first monomer includes at least one of phosphorylcholine analogous group-containing compounds, and the second monomer includes at least one of quaternary ammonium cations containing an epoxy group and an aryl group. Further, the graft copolymer of the present invention may additionally include other monomers in addition to the first monomer and the second monomer.

### Polysaccharides

Polysaccharide in the copolymer of the present invention constitutes a main chain. The polysaccharide included in the copolymer of the present invention may be used without limitation as long as the polysaccharide constitutes the main chain. For example, cellulose and a derivative thereof, starch and a derivative thereof, various plant gums and the like may be used, and specific examples thereof include cellulose, hydroxyethyl cellulose, hydroxypropyl cellulose, methyl cellulose, ethyl cellulose, carboxymethyl cellulose, carboxymethyl hydroxyethyl cellulose, guar gum, locust bean gum, xanthan gum, galactomannon gum, starch derived from corn, potato, wheat, rice, and the like, amylose, and the like, and may include C1 to C6 alkyl derivatives thereof. These may be used either alone or as a mixture thereof. The type of polysaccharide is not particularly limited, but cellulose and a derivative thereof are preferred from the viewpoint of moisture retention, biocompatibility and the like, and hydroxyethyl cellulose is more preferred in terms of moisture retention.

### First monomer

A first monomer in the copolymer of the present invention constitutes a side chain. The first monomer included in the copolymer of the present invention consists of at least one of phosphorylcholine analogous group-containing compounds represented by the following Formula (1).

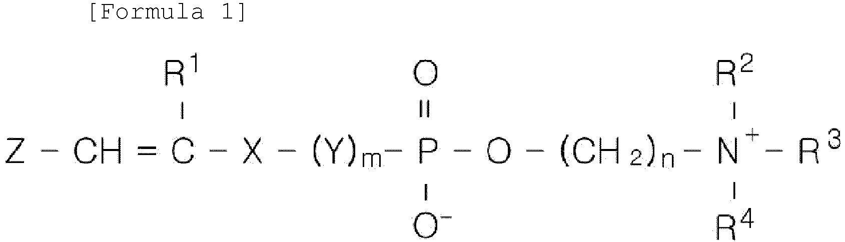

In Formula (1), X represents a divalent organic residue, Y represents an alkyleneoxy group having from 1 to 6 carbon atoms, and Z represents a hydrogen atom or R⁵-O-CO- (here, R⁵ represents an alkyl group having from 1 to 10 carbon atoms, or a hydroxyalkyl group having from 1 to 10 carbon atoms). Further, R¹ represents a hydrogen atom or a methyl group, R², R³ and R⁴ are the same or different groups and represent a hydrogen atom and a hydrocarbon group or a hydroxyhydrocarbon group having from 1 to 6 carbon atoms. m is 0 or 1, and n is an integer from 2 to 4.

Examples of the phosphorylcholine analogous group-containing compound represented by Formula (1) include 2-((meth)acryloyloxy)ethyl-2'-(trimethylammonio)ethylphosphate, 3-((meth)acryloyloxy)propyl-2'-(trimethylammonio)ethylphosphate, 4-((meth)acryloyloxy)butyl-2'-(trimethylammonio)ethylphosphate, 5-((meth)acryloyloxy)pentyl-2'-(trimethylammonio)ethylphosphate, 6-((meth)acryloyloxy)hexyl-2'-(trimethylammonio)ethylphosphate, 2-((meth)acryloyloxy)ethyl-2'-(triethylammonio)ethylphosphate, 2-((meth)acryloyloxy)ethyl-2'-(tripropylammonio)ethylphosphate, 2-((meth)acryloyloxy)ethyl-2'-(tributylammonio)ethylphosphate, 2-((meth)acryloyloxy)propyl-2'-(trimethylammonio)ethylphosphate, 2-((meth)acryloyloxy)butyl-2'-(trimethylammonio)ethylphosphate, 2-((meth)acryloyloxy)pentyl-2'-(trimethylammonio)ethylphosphate, 2-((meth)acryloyloxy)hexyl-2' - (trimethylammonio)ethylphosphate, 2-(vinyloxy)ethyl-2'-(trimethylammonio)ethylphosphate, 2-(allyloxy)ethyl-2'-(trimethylammonio)ethylphosphate, 2-(p-vinylbenzyloxy)ethyl-2'-(trimethylammonio)ethylphosphate, 2-(p-vinylbenzoyloxy)ethyl-2'-(trimethylammonio)ethylphosphate, 2-(stylyloxy)ethyl-2'-(trimethylammonio)ethylphosphate, 2-(p-vinylbenzyl)ethyl-2'-(trimethylammonio)ethylphosphate, 2-(vinyloxycarbonyl)ethyl-2'-(trimethylammonio)ethylphosphate, 2-(allyloxycarbonyl)ethyl-2'-(trimethylammonio)ethylphosphate, 2-(acryloylamino)ethyl-2'-(trimethylammonio)ethylphosphate, 2-(vinylcarbonylamino)ethyl-2'-(trimethylammonio)ethylphosphate, 2-(allyloxycarbonylamino)ethyl-2'-(trimethylammonio)ethylphosphate, 2-(butyroyloxy)ethyl-2'-(trimethylammonio)ethylphosphate, 2-(crotonoyloxy)ethyl-2'-(trimethylammonio)ethylphosphate, ethyl-(2'-trimethylammonioethylphosphorylethyl)fumarate, butyl-(2'-trimethylammonioethylphosphorylethyl)fumarate, hydroxyethyl-(2'-trimethylammonioethylphosphorylethyl)fumarate, ethyl-(2'-trimethylammonioethylphosphorylethyl)fumarate, butyl-(2'-trimethylammonioethylphosphorylethyl)fumarate, hydroxyethyl-(2'-trimethylammonioethylphosphorylethyl)fumarate, and the like. The type of phosphorylcholine analogous group-containing compound is not particularly limited, but 2-(methacryloyloxy)ethyl-2'-(trimethylammonio)ethylphosphate (also referred to as 2-methacryloyloxyethylphosphorylcholine, and hereinafter, abbreviated as MPC) is preferred from the viewpoint of availability and biocompatibility. These may be used either alone or as a mixture thereof. The "((meth)acryloyloxy)ethyl" and the like in the phosphorylcholine analogous group-containing compound mean all of acryloyloxyethyl, methacryloyloxyethyl, and the like.

The content of the first monomer in the copolymer of the present invention is not particularly limited, but when biocompatibility, moisture retention, and compatibility with the second monomer are considered, the content is preferably from 0.5 part by weight to 80 parts by weight, more preferably from 1 part by weight to 60 parts by weight, and most preferably from 5 parts by weight to 40 parts by weight, based on 100 parts by weight of the polysaccharide.

### Second monomer

A second monomer in the copolymer of the present invention constitutes a side chain. The second monomer included in the copolymer of the present invention consists of at least one of quaternary ammonium compounds containing an epoxy group, and the quaternary ammonium compound containing an epoxy group preferably does not contain a phosphorylcholine analogous group represented by Formula (1) to be described below. The quaternary ammonium compound containing an epoxy group serves to enhance the solubility of the copolymer and facilitate the adsorption of the copolymer to hair, and enhances a hair conditioning effect of the copolymer. The type of second monomer is not particularly limited, and when the conditioning effect of the copolymer, commercial availability thereof, and the like are considered, epoxy alkyl trialkyl ammonium halide is preferred. At this time, the alkyl group of the epoxy alkyl has from 2 to 20 carbon atoms, the alkyl group of the trialkyl has from 1 to 20 carbon atoms, and the alkyl groups more preferably have the same or different carbon atoms. In addition, the second monomer is most preferably epoxypropyl trimethyl ammonium chloride.

The content of the second monomer in the copolymer of the present invention is not particularly limited, but when conditioning effect of the copolymer and compatibility with the first monomer are considered, the content is preferably from 5 parts by weight to 95 parts by weight, more preferably from 10 parts by weight to 80 parts by weight, and most preferably from 20 parts by weight to 60 parts by weight, based on 100 parts by weight of the polysaccharide.

### Other monomers

Meanwhile, the copolymer of the present invention may additionally include other monomers as a side chain in addition to the aforementioned first monomer and second monomer, depending on cases. The types of other monomers are not particularly limited, but a monomer that may be dissolved in an aqueous alcohol or an alcohol having 4 or less carbon atoms is preferred in terms of biocompatibility and moisture retention. Examples thereof include a diallyl dialkyl ammonium salt, acrylic acid, methacrylic acid, acryl amide, methacryl amide, mono- or di-N-substituted acrylamide and methacrylamide, acrylonitrile, vinyl pyrrolidone, sodium or ammonium styrene sulfonate, hydroxyalkyl acrylate, methacrylate, and the like, but are not always limited thereto. These may be used either alone or as a mixture thereof.

More preferably, a diallyl dialkyl ammonium salt is included as other monomers. At this time, the alkyl in the diallyl dialkyl ammonium salt has from 1 to 12 carbon atoms, and the purity thereof is not particularly limited. Furthermore, the allyl dialkyl ammonium salt is preferably a diallyl dialkyl ammonium halide, and more preferably chloride or bromide. Specific examples thereof include N,N-diallyl-N,N-dimethylammonium chloride, N,N-diallyl-N,N-diethylammonium chloride, N,N-diallyl-N-methyl-N-dodecylammonium chloride, N,N-diallyl-N-methyl-N-butylammonium chloride, N,N-diallyl-N-methyl-N-octylammonium chloride, N,N-diallyl-N-methyl-N-decylammonium chloride, N,N-diallyl-N,N-dimethylammonium bromide, N,N-diallyl-N,N-diethylammonium bromide, N,N-diallyl-N-methyl-N-dodecylammonium bromide, N,N-diallyl-N-methyl-N-butylammonium bromide, N,N-diallyl-N-methyl-N-octylammonium bromide, N,N-diallyl-N-methyl-N-decylammonium bromide, and the like.

The content of other monomers except for the first monomer and the second monomer in the copolymer of the present invention is not particularly limited, but when biocompatibility, moisture retention, and compatibility with the first monomer or the second monomer are considered, the content is preferably from 0.5 part by weight to 50 parts by weight, more preferably from 1 part by weight to 40 parts by weight, and most preferably from 1.5 parts by weight to 20 parts by weight, based on 100 parts by weight of the polysaccharide.

When the copolymer of the present invention includes the polysaccharide and the first monomer without including the second monomer, it is preferred that the final copolymer is constituted to include from 30% by weight to 99.5% by weight of the polysaccharide and from 0.5% by weight to 70% by weight of the phosphorylcholine analogous group-containing monomer. However, when the copolymer of the present invention additionally includes monomers other than the above-described two ingredients, the copolymer of the present invention may include, for example, from 30% by weight to 99.5% by weight of the polysaccharide, from 0.45% by weight to 69.95% by weight of the phosphorylcholine analogous group-containing monomer, and from 0.05% by weight to 69.55% by weight of other monomers. At this time, when the copolymer of the present invention includes each of the constitutional ingredients within the aforementioned range, the range is preferred in that the impression from use, such as hair moisture retention, flexibility, voluminousness, and the like is enhanced.

Furthermore, it is preferred that the molecular weight of the copolymer of the present invention usually ranges from 10,000 to 2,000,000 as a weight average molecular weight. The content of nitrogen in the copolymer of the present invention is not particularly limited, but is preferably from 0.2% to 2.5% in terms of moisture retention, flexibility, and the like. The viscosity of a 1% aqueous solution of the copolymer of the present invention is not particularly limited, but may be, for example, from 10 cPs to 2,000 cPs, and more preferably from 30 cPs to 1,500 cPs. When the copolymer of the present invention satisfies the above-described constitutional ingredients and the range thereof, the range is preferred in that the impression from use, such as skin and hair moisture retention, skin affinity, flexibility, hair voluminousness, and the like, is enhanced.

### Preparation of new polysaccharide-based copolymer

A new polysaccharide-based copolymer according to the present invention may be prepared by a method of simultaneously subjecting a first monomer and a second monomer to graft bonding to a polysaccharide, and by a method of first subjecting the second monomer to graft bonding to the polysaccharide and then subjecting the first monomer to graft bonding to the polysaccharide. Hereinafter, a method of preparing a copolymer by sequentially subjecting a second monomer and a first monomer to graft bonding to a polysaccharide will be described.

First, alcohol and water are introduced into a reactor, polysaccharide and a second monomer are introduced thereinto, and then the mixture is dissolved while being stirred at about 20°C to 30°C. Thereafter, a basic aqueous solution is introduced into the reactor and stirred, and then the mixture is warmed to about 40°C to 70°C. The mixture is subjected to graft bonding reaction while maintaining the warmed temperature, and then the graft bonding reaction is terminated by introducing an acidic aqueous solution thereto. After the graft reaction is terminated, a reaction product is diluted by introducing alcohol and distilled water into the reactor and stirring the mixture, and a polysaccharide-based copolymer to which the second monomer is graft-bonded is obtained by filtering, washing, and drying the diluted reaction product.

A usual radical polymerization method and the like may be used in the presence of a solvent and an emulsifying agent in order to graft-bond the first monomer to the polysaccharide to which the second monomer is graft-bonded. For example, it is possible to refer to preparation methods described in US Patent Nos. 4,131,576 and 4,464,523, and the like, but the methods will be described in detail as follows. The solvent and the emulsifying agent are added to the reactor, a phosphorylcholine analogous group-containing compound and water are added thereto, a free radical polymerization catalyst is added thereto, a polysaccharide to which the second monomer is graft-bonded is subsequently added thereto, and then a new polysaccharide-based copolymer may be prepared by warming the mixture to 40°C to 100°C to be subjected to polymerization reaction for 30 minutes to 72 hours. Preferably, a final product may be obtained by washing the recovered copolymer again in the reactor with an appropriate solvent, for example, isopropanol, and the like once to several times, and then filtering and drying the mixture, and pulverizing and packaging the mixture. At this time, the copolymer is prepared preferably under the atmosphere of an inert gas such as nitrogen, argon, and the like. The solvent is not particularly limited, but a solvent, which may disperse a polysaccharide to which the second monomer is graft-bonded, is preferred, and specifically, ethanol, isopropanol, butanol, isoparaffin, toluene, mineral oil, and the like may be used either alone or as a mixture thereof. The emulsifying agent is not particularly limited as long as the emulsifying agent is a usual emulsifying agent, and specific examples thereof include hydroxyethylated nonyl phenols, hydroxyethylated long-chain monocarboxylic acids and fatty acids, fatty acid esters of sorbitol, hydroxyethylated fatty acid esters of sorbitol, linear alkyl sulfonates and sulfates such as alkyl aryl sulfonate, lauryl sulfonate, or sulfosuccinic acid ester, and the like. Examples of a specific product name of the emulsifying agent include Span 60 and 80 (Hercules, Inc.), Brij 92 (Hercules, Inc.), Igepal 990 (GAF), Triton X (Rohm and Haas Co.), Calsoft 30 (Pilot Chemical Co.), and the like. The free radical polymerization catalyst is not particularly limited as long as the free radical polymerization catalyst is a usual radical polymerization catalyst, but preferably, (NH₄)₂S₂O₈, K₂S₂O₈, Na₂S₂O₈, an azo compound, a Redox initiator, water, and the like may be used.

### Composition 1 for hair care

Another aspect of the present invention relates to a composition for hair care which includes the above-described new polysaccharide-based graft copolymer 1 as an active ingredient. The content of the polysaccharide-based graft copolymer in the composition for hair care according to the present invention is not particularly limited, but when the impression from use, moisture-retaining feeling, and compatibility with other constitutional ingredients are considered, the content is preferably from 0.01% by weight to 5% by weight, and more preferably from 0.1% by weight to 4% by weight, based on the total weight of the composition.

The composition 1 for hair care of the present invention includes the above-described polysaccharide-based graft copolymer to which the first monomer and the second monomer are graft-bonded as an active ingredient. The quaternary ammonium cation containing an epoxy group imparts excellent hair adsorptivity and the phosphorylcholine analogous group-containing compound imparts excellent biocompatibility and superior moisture retention, and thus a composition for hair care, which includes the copolymer of the present invention in which the same are introduced into a polysaccharide, also exhibits enhanced biocompatibility and solubility and significantly improved moisturizing capacity, protecting effect of damaged hair, and the like, compared to a composition for hair care in the related art which includes a polysaccharide-containing copolymer into which other monomers are introduced.

The composition 1 for hair care of the present invention may include one or more of usual additives, such as an appropriate carrier for the aforementioned graft copolymer, a surfactant, an aroma ingredient, an opacifier, a combining aid, protein, an aerosol propellent, a thickener, a gelling agent, and the like.

Further, the composition 1 for hair care of the present invention is not particularly limited with respect to the dosage form thereof, and the composition 1 may be prepared in various dosage forms including, for example, shampoo, rinse, wax for fixing hair, conditioner for hair care, and the like. In addition, the composition of the present invention may be used in a product for skin care in addition to the above-described products, and may impart moisture retaining and skin protecting effects.

### Composition 2 for skin care

A composition 2 for skin care of the present invention includes the new polysaccharide-based graft copolymer as an active ingredient, and the composition for skin care is not particularly limited with respect to the content thereof, but includes the above-described copolymer in an amount from 0.01% by weight to 2% by weight, and preferably from 0.05% by weight to 0.5% by weight. When the copolymer is included within the range, the composition is advantageous in terms of impression from use, moisture-retaining feeling, skin protection, and the like. The new graft copolymer is a graft copolymer including a polysaccharide and a phosphorylcholine analogous group-containing monomer. The phosphorylcholine analogous group-containing monomer imparts excellent biocompatibility and superior moisture retention, and thus a composition for skin care, which includes the copolymer of the present invention in which the same monomer is introduced into a polysaccharide, also exhibits enhanced biocompatibility and solubility and significantly improved moisturizing capacity, protecting effect of damaged skin, and the like, compared to a composition for skin care in the related art which includes a polysaccharide-containing copolymer into which other monomers are introduced.

The composition 2 for skin care of the present invention may include one or more of usual additives, such as an appropriate carrier for the aforementioned graft copolymer, a surfactant, an aroma ingredient, an opacifier, a combining aid, protein, an aerosol propellent, a thickener, a gelling agent, and the like.

Furthermore, the composition 2 for skin care of the present invention is not particularly limited with respect to the dosage form thereof, and may be prepared in various dosage forms including, for example, milk lotion, cream, toner, conditioner for skin care, and the like. Further, the composition of the present invention may be used in a product for hair care in addition to the above-described products, and may impart moisture retaining and skin protecting effects.

Hereinafter, the present invention will be described in more detail through the Examples. However, the following Examples are provided to describe the present invention more clearly, and not intended to limit the protection scope of the present invention.

### [Preparation of polysaccharide-based graft copolymer 1]

Preparation Example 1 (Preparation of polysaccharide-based graft copolymer 1 to which a quaternary ammonium compound containing an epoxy group and a phosphorylcholine analogous group-containing compound are bonded)

250 g of isopropyl alcohol and 25 g of distilled water were introduced into a reactor and mixed while being stirred, and then an MPC solution in which 8 g of MPC had been dissolved in 3 g of distilled water was introduced into the reactor while being stirred. Thereafter, 0.102 g of EDTA-2Na as a pH adjuster and 0.2 g of azobisisobutyronitrile (AIBN) as a catalyst were introduced into the reactor, and the mixture was stirred for about 20 minutes. Thereafter, 40 g of the polysaccharide-based graft copolymer to which 2,3-epoxypropyl trimethyl ammonium chloride was bonded in Preparation Example 1 was introduced into the reactor, nitrogen substitution was performed for about 10 minutes, the temperature of the reactor was increased to about 70°C, and the temperature was maintained at 70°C ± 2°C for 6 hours to subject the mixture to graft-bonding reaction. At this time, nitrogen substitution was initiated 10 minutes before the increase in temperature, and was maintained until the reaction was completed. After the reaction was completed, the reaction product was filtered, washed with a 90% isopropyl alcohol aqueous solution again, re-filtered, and then dried to obtain a polysaccharide-based graft copolymer to which 2,3-epoxypropyl trimethyl ammonium chloride and MPC are bonded. At this time, the obtained final copolymer had a nitrogen content of 2.0%, and the 1% aqueous solution had a viscosity of about 1,100 cPs.

### Comparative Preparation Example 1 (Preparation of polysaccharide-based graft copolymer to which a quaternary ammonium compound containing an epoxy group is bonded)

1,489 g of isopropyl alcohol and 165.5 g of distilled water were introduced into a reactor and mixed while being stirred, 450 g of hydroxyethyl cellulose was introduced thereinto, and the mixture was stirred at about 25°C for 10 minutes. Thereafter, 185 g of 2,3-epoxypropyl trimethyl ammonium chloride was introduced thereinto, the mixture was stirred for 15 minutes, 57 g of a sodium hydroxide aqueous solution having a concentration of about 10% was introduced into the reactor for 4 minutes, and the resulting mixture was stirred for 20 minutes. Thereafter, the temperature of the reactor was increased to 50°C to 55°C and maintained for about 6 hours to react the mixture, and 17.5 g of glacial acetic acid was introduced to terminate the reaction. Thereafter, 1,000 g of a 90% isopropyl alcohol aqueous solution was introduced into the reactor to dilute a reaction product, the diluted reaction product was filtered, washed with a 90% isopropyl alcohol aqueous solution again, re-filtered, and dried to obtain a polysaccharide-based polymer to which 2,3-epoxypropyl trimethyl ammonium chloride was bonded.

### [Preparation of polysaccharide-based graft copolymer 2] Comparative Preparation Example 2

126 g of Isopar E, 1.95 g of Span-80 and 1.95 g of Triton X-100 as emulsifying agents were introduced into a 250 ML reactor, 0.012 g of EDTA-2Na and 1.9 g of Na₂HPO₄ as pH adjusters were added thereto, and 15 g of diallyl dimethyl ammonium chloride (purity 63% and hereinafter, abbreviated as DADMAC) was introduced thereto. 0.26 g of (NH₄)₂S₂O₈ as a catalyst and 5.2 g of H₂O were dissolved and the solution was put thereto, and subsequently, 40 g of hydroxyethyl cellulose (hereinafter, abbreviated as HEC) was added thereto to subject the mixture to polymerization reaction. The product was warmed to 70°C for 1 hour, and then was polymerized at 68°C to 72°C for 6 hours. At this time, nitrogen substitution was initiated 30 minutes before the increase in temperature, and was maintained until the reaction was completed. The polymerization solution was filtered and vacuum dried at room temperature for 48 hours to obtain a copolymer. The obtained copolymer was secondly washed at 45°C for 2 hours using 63.3 g of isopropanol in the 250 ML reactor, and then filtered, dried, and ground with a 20 mesh screen to obtain a final copolymer. At this time, the obtained final copolymer had a nitrogen content of 1.5%, and the 1% aqueous solution had a viscosity of 500 cPs.

### Preparation Example 2

A polysaccharide-based graft copolymer to which MPC and DADMAC were bonded was obtained in the same manner as in Comparative Preparation Example 2, except that an MPC solution in which 15 g of DADMAC and 8 g of MPC had been dissolved in 10 g of distilled water was used instead of 15 g of DADMAC. At this time, the obtained final copolymer had a nitrogen content of 2.0%, and the 1% aqueous solution had a viscosity of 300 cPs.

### Preparation Example 3

A polysaccharide-based graft copolymer to which MPC, DADMAC, and 2-HEA were bonded was obtained in the same manner as in Comparative Preparation Example 2, except that an MPC solution in which 15 g of DADMAC, 8 g of MPC, and 4 g of 2-hydroxy ethyl acrylate (hereinafter, abbreviated as 2-HEA) had been dissolved in 10 g of distilled water was used instead of 15 g of DADMAC. At this time, the obtained final copolymer had a nitrogen content of 1.95%, and the 1% aqueous solution had a viscosity of 350 cPs.

### Preparation Example 4

A polysaccharide-based graft copolymer to which MPC, DADMAC, and SA were bonded was obtained in the same manner as in Comparative Preparation Example 2, except that an MPC solution in which 15 g of DADMAC, 8 g of MPC, and 4 g of stearyl acrylate (hereinafter, abbreviated as SA) had been dissolved in 10 g of distilled water was used instead of 15 g of DADMAC. At this time, the obtained final copolymer had a nitrogen content of 1.97%, and the 1% aqueous solution had a viscosity of 550 cPs.

### Comparative Preparation Example 3

A polysaccharide-based graft copolymer to which DADMAC and 2-HEA were bonded was obtained in the same manner as in Preparation Example 3, except that a solution in which 15 g of DADMAC and 4 g of 2-HEA had been dissolved in 10 g of distilled water was used instead of an MPC solution in which 15 g of DADMAC, 8 g of MPC, and 4 g of 2-HEA had been dissolved in 10 g of distilled water. At this time, the obtained final copolymer had a nitrogen content of 1.45%, and the 1% aqueous solution had a viscosity of 300 cPs.

### Comparative Preparation Example 4

A polysaccharide-based graft copolymer to which DADMAC and SA were bonded was obtained in the same manner as in Preparation Example 4, except that a solution in which 15 g of DADMAC and 4 g of SA had been dissolved in 10 g of distilled water was used instead of the MPC solution in which 15 g of DADMAC, 8 g of MPC, and 4 g of SA had been dissolved in 10 g of distilled water. At this time, the obtained final copolymer had a nitrogen content of 1.52%, and the 1% aqueous solution had a viscosity of 500 cPs.

### [Preparation of polysaccharide-based graft copolymer 3]

### Preparation Example 5

250 g of isopropyl alcohol and 25 g of distilled water were introduced into a reactor and mixed while being stirred, and then an MPC solution in which 12g of MPC had been dissolved in 6 g of distilled water was introduced into the reactor while being stirred. Thereafter, 0.102 g of EDTA-2Na as a pH adjuster and 0.2 g of azobisisobutyronitrile (AIBN) as a catalyst were introduced into the reactor, and the mixture was stirred for about 20 minutes. Thereafter, 40 g of HEC was introduced into the reactor and nitrogen substitution was performed for about 10 minutes, the temperature of the reactor was increased to about 70°C, and the temperature was maintained at 70°C ± 2°C for 6 hours to subject the mixture to graft-bonding reaction. At this time, nitrogen substitution was initiated 10 minutes before the increase in temperature, and was maintained until the reaction was completed. After the reaction was completed, the reaction product was filtered, washed with a 90% isopropyl alcohol aqueous solution again, re-filtered, and then dried to obtain a polysaccharide-based graft copolymer to which MPC is bonded. At this time, the obtained final copolymer had had a nitrogen content of 1.4%, and the 1% aqueous solution had a viscosity of about 600 cPs.

### Preparation Example 6

A polysaccharide-based graft copolymer to which MPC and 2-HEA were bonded was obtained in the same manner as in Preparation Example 5, except that a solution in which 12 g of MPC and 4 g of 2-HEA had been dissolved in water was used instead of the MPC solution in which 12 g of MPC had been dissolved in 6 g of distilled water in Preparation Example 5. At this time, the obtained final copolymer had a nitrogen content of 1.35%, and the 1% aqueous solution had a viscosity of 550 cPs.

### Preparation Example 7

A polysaccharide-based graft copolymer to which MPC and stearyl acrylate were bonded was obtained in the same manner as in Preparation Example 5, except that a solution in which 12 g of MPC and 4 g of stearyl acrylate had been dissolved in water was used instead of the MPC solution in which 12 g of MPC had been dissolved in 6 g of distilled water in Preparation Example 5. At this time, the obtained final copolymer had a nitrogen content of 1.37%, and the 1% aqueous solution had a viscosity of 750 cPs.

### Comparative Preparation Example 5

A polysaccharide-based graft copolymer to which 2-HEA were bonded was obtained in the same manner as in Preparation Example 5, except that a solution in which 4 g of 2-HEA had been dissolved in 6 g of distilled water was used instead of the MPC solution in which 12 g of MPC had been dissolved in 6 g of distilled water in Preparation Example 5. At this time, the obtained final copolymer had a nitrogen content of 0.10%, and the 1% aqueous solution had a viscosity of 2,000 cPs.

### Comparative Preparation Example 6

A polysaccharide-based graft copolymer to which stearyl acrylate were bonded was obtained in the same manner as in Preparation Example 5, except that 4 g of stearyl acrylate was used instead of the MPC solution in which 12 g of MPC had been dissolved in 6 g of distilled water in Preparation Example 5. At this time, the obtained final copolymer had a nitrogen content of 0.1%, and the 1% aqueous solution had a viscosity of 2,750 cPs.

### [Preparation of composition for hair care]

### Example 1

40.05 g of water was put into a mixing bath, 0.8 g of the copolymer prepared in Preparation Example 1 was put thereinto while increasing the temperature, and then 1 g of propylene glycol was put thereinto, and the temperature was increased to 75°C.

18 g of sodium lauryl sulfate, 17 g of sodium laureth sulfate, 2.5 g of cocofatty acid diethanolamide, 8 g of cocamido propyl betain, 3 g of soyamidopropylamine oxide, 0.5 g of cetearyl alcohol, 2 g of disodium cocoamphodiacetate, 0.6 g of ethylene glycol distearate, 2 g of polyglyceryl-2 caprate, 0.5 g of cyclopentasiloxane and dimethiconol, 1 g of dimethicone, and 0.05 g of tetrasodium EDTA were introduced into the mixing bath, and mixed at 75°C for approximately 30 minutes until the mixture became uniform, and then the mixture was cooled to 45°C.

Subsequently, 0.05 g of alantion, 0.05 g of methyl chloroisothiazolinone & methyl isothiazolinone, 0.1 g of DL-panthenol, 0.5 of citric acid, and 0.3 g of fragrance were introduced into the mixing bath, and mixed at 45°C until the mixture became uniform to prepare 100 g of the composition for hair care of the present invention.

### Example 2

A composition for hair care was prepared in the same manner as in Example 1, except that the copolymer prepared in Preparation Example 2 was used.

### Example 3

A composition for hair care was prepared in the same manner as in Example 1, except that the copolymer prepared in Preparation Example 3 was used.

### Example 4

A composition for hair care was prepared in the same manner as in Example 1, except that the copolymer prepared in Preparation Example 4 was used.

### Comparative Example 1

A composition for hair care was prepared in the same manner as in Example 1, except that the copolymer prepared in Comparative Preparation Example 1 was used.

### Comparative Example 2

A composition for hair care was prepared in the same manner as in Example 1, except that the copolymer prepared in Comparative Preparation Example 2 was used.

### Comparative Example 3

A composition for hair care was prepared in the same manner as in Example 1, except that the copolymer prepared in Comparative Preparation Example 3 was used.

### Comparative Example 4

A composition for hair care was prepared in the same manner as in Example 1, except that the copolymer prepared in Comparative Preparation Example 4 was used.

### [Protecting effect test of damaged hair by composition 1 for hair care]

Protecting effects of hair were tested through a panel test of each of the compositions for hair care prepared in Examples 1 to 4 and Comparative Examples 1 to 4 with respect to feeling while hair is washed, combing in a wet state, combing after hair is dried, voluminousness of hair, gloss of hair, and the like. The results are shown in the following Tables 1 to 5.

**[Table 1]**

| Panel | Example 1 | Example 2 | Example 3 | Example 4 | Comparative Example 1 | Comparative Example 2 | Comparative Example 3 | Comparative Example 4 |
|---|---|---|---|---|---|---|---|---|
| PJH | 10 | 10 | 10 | 10 | 8 | 5 | 5 | 5 |
| MJL | 10 | 10 | 10 | 10 | 8 | 5 | 5 | 5 |
| LSY | 8 | 8 | 8 | 8 | 5 | 8 | 5 | 8 |
| LMI | 10 | 10 | 10 | 10 | 8 | 8 | 3 | 8 |
| SSM | 10 | 10 | 10 | 10 | 5 | 5 | 5 | 5 |
| PYS | 8 | 8 | 10 | 10 | 5 | 8 | 5 | 5 |
| HYA | 10 | 10 | 10 | 10 | 8 | 8 | 8 | 5 |
| KJH | 10 | 8 | 8 | 10 | 5 | 5 | 5 | 5 |
| YSS | 8 | 10 | 10 | 8 | 5 | 8 | 3 | 3 |
| KJK | 10 | 10 | 10 | 10 | 5 | 3 | 3 | 3 |

| | | | | | | | | |
|---|---|---|---|---|---|---|---|---|
| Feeling while hair is washed (10: Very good/ 8: Good/ 5: Normal/ 3: Bad/ 1: Very bad) | | | | | | | | |

**[Table 2]**

| Panel | Example1 | Example 2 | Example 3 | Example 4 | Comparative Example 1 | Comparative Example 2 | Comparative Example 3 | Comparative Example 4 |
|---|---|---|---|---|---|---|---|---|
| PJH | 10 | 10 | 10 | 10 | 8 | 5 | 8 | 5 |
| MJL | 8 | 10 | 10 | 10 | 5 | 5 | 5 | 5 |
| LSY | 8 | 8P | 8 | 10 | 5 | 5 | 5 | 5 |
| LMI | 10 | 8 | 10 | 10 | 5 | 8 | 5 | 5 |
| SSM | 10 | 10 | 10 | 10 | 5 | 8 | 5 | 5 |
| PYS | 8 | 10 | 10 | 10 | 5 | 8 | 5 | 5 |
| HYA | 10 | 10 | 8 | 10 | 8 | 8 | 8 | 5 |
| KJH | 8 | 8 | 8 | 10 | 5 | 5 | 5 | 5 |
| YSS | 10 | 10 | 10 | 8 | 5 | 3 | 3 | 3 |
| KJK | 10 | 10 | 10 | 10 | 8 | 3 | 3 | 3 |

| | | | | | | | | |
|---|---|---|---|---|---|---|---|---|
| Combing in a wet state (10: Very good/ 8: Good/ 5: Normal/ 3: Bad/ 1: Very bad) | | | | | | | | |

**[Table 3]**

| Panel | Example 1 | Example 2 | Example 3 | Example 4 | Comparative Example 1 | Comparative Example 2 | Comparative Example 3 | Comparative Example 4 |
|---|---|---|---|---|---|---|---|---|
| PJH | 10 | 8 | 10 | 10 | 5 | 5 | 5 | 5 |
| MJL | 8 | 10 | 10 | 10 | 5 | 5 | 3 | 5 |
| LSY | 10 | 10 | 10 | 10 | 8 | 5 | 5 | 8 |
| LMI | 10 | 8 | 10 | 8 | 5 | 3 | 3 | 5 |
| SSM | 10 | 10 | 10 | 10 | 5 | 5 | 5 | 5 |
| PYS | 8 | 10 | 10 | 10 | 5 | 5 | 5 | 5 |
| HYA | 10 | 10 | 10 | 10 | 5 | 8 | 8 | 8 |
| KJH | 10 | 8 | 8 | 10 | 3 | 3 | 5 | 5 |
| YSS | 10 | 10 | 10 | 8 | 5 | 3 | 3 | 5 |
| KJK | 8 | 10 | 10 | 10 | 8 | 3 | 3 | 3 |

| | | | | | | | | |
|---|---|---|---|---|---|---|---|---|
| Combing after hair is dried (10: Very good/ 8: Good/ 5: Normal/ 3: Bad/ 1: Very bad) | | | | | | | | |

**[Table 4]**

| Panel | Example 1 | Example 2 | Example 3 | Example 4 | Comparative Example 1 | Comparative Example 2 | Comparative Example 3 | Comparative Example 4 |
|---|---|---|---|---|---|---|---|---|
| PJH | 8 | 10 | 10 | 10 | 5 | 5 | 5 | 5 |
| MJL | 8 | 10 | 10 | 10 | 5 | 5 | 5 | 5 |
| LSY | 10 | 10 | 10 | 10 | 8 | 8 | 5 | 5 |
| LMI | 8 | 8 | 8 | 8 | 5 | 3 | 3 | 5 |
| SSM | 10 | 10 | 10 | 10 | 5 | 8 | 5 | 5 |
| PYS | 8 | 10 | 10 | 10 | 5 | 5 | 5 | 5 |
| HYA | 10 | 10 | 10 | 10 | 5 | 8 | 5 | 8 |
| KJH | 8 | 8 | 10 | 10 | 3 | 5 | 5 | 5 |
| YSS | 10 | 10 | 10 | 10 | 5 | 8 | 5 | 5 |
| KJK | 8 | 8 | 10 | 10 | 8 | 5 | 3 | 5 |

| | | | | | | | | |
|---|---|---|---|---|---|---|---|---|
| Voluminousness of hair (10: Very good/ 8: Good/ 5: 5 Normal/ 3: Bad/ 1: Very bad) | | | | | | | | |

**[Table 5]**

| Panel | Example 1 | Example 2 | Example 3 | Example 4 | Comparative Example 1 | Comparative Example 2 | Comparative Example 3 | Comparative Example 4 |
|---|---|---|---|---|---|---|---|---|
| PJH | 10 | 10 | 10 | 10 | 5 | 5 | 5 | 8 |
| MJL | 8 | 10 | 10 | 10 | 5 | 5 | 5 | 5 |
| LSY | 10 | 10 | 10 | 10 | 5 | 5 | 5 | 8 |
| LMI | 8 | 8 | 8 | 10 | 5 | 3 | 3 | 5 |
| SSM | 10 | 10 | 10 | 10 | 5 | 3 | 5 | 5 |
| PYS | 10 | 8 | 10 | 10 | 5 | 5 | 5 | 5 |
| HYA | 10 | 10 | 10 | 10 | 5 | 5 | 5 | 5 |
| KJH | 8 | 8 | 10 | 10 | 3 | 5 | 3 | 5 |
| YSS | 10 | 10 | 10 | 10 | 5 | 5 | 5 | 5 |
| KJK | 8 | 8 | 10 | 10 | 3 | 5 | 5 | 5 |

| | | | | | | | | |
|---|---|---|---|---|---|---|---|---|
| Gloss of hair (10: Very good/ 8: Good/ 5: Normal/ 3: Bad/ 1: Very bad) | | | | | | | | |

### [Preparation of composition for skin care]

### Example 5

81.91 g of water was put into a mixing bath, 0.25 g of the copolymer prepared in Preparation Example 5 was put thereinto while increasing the temperature, and then 10g of 1,3-butylene glycol was put thereinto, and the temperature was increased to 75°C.

0.5 g of methyl glucoside (EO 10), 0.5 g of dipropylene glycol, 0.3 g of glycerine glycerylacrylate/acrylic acid copolymer, 2 g of zantan gum (2%), 0.02 g of disodium EDTA, 0.1 g of methyl paraben, 0.15 g of phenoxy ethanol, 0.02 g of allantoin, 0.2 g of DL-panthenol, and 0.4 g of PEG-40 hydrogenated caster oil were introduced into the mixing bath, and mixed at 75°C for approximately 30 minutes until the mixture became uniform, and then the mixture was cooled to 45°C.

Subsequently, 3.5 of ethyl alcohol and 0.15 g of fragrance were introduced into the mixing bath, and mixed at 45°C until the mixture became uniform to prepare 100 g of the composition for skin care of the present invention.

### Example 6

A composition for skin care was prepared in the same manner as in Example 5, except that the copolymer prepared in Preparation Example 6 was used.

### Example 7

A composition for skin care was prepared in the same manner as in Example 5, except that the copolymer prepared in Preparation Example 7 was used.

### Comparative Example 5

A composition for skin care was prepared in the same manner as in Example 5, except that the copolymer prepared in Comparative Preparation Example 5 was used.

### Comparative Example 6

A composition for skin care was prepared in the same manner as in Example 5, except that the copolymer prepared in Comparative Preparation Example 6 was used.

### Comparative Example 7

A composition for skin care was prepared in the same manner as in Example 5, except that HEC was used instead of the copolymer prepared.

### [Effect test of composition for skin care]

If sodium dodecyl sulfate that is an anionic surfactant is applied to the skin every day, irritation to the skin is caused, and the skin becomes dry and erythema is produced on the skin due to the irritation, moisture is lost due to reduction in the skin barrier function, and an increase ratio in skin moisture, erythema, and a percutaneous moisture loss amount were tested in order to confirm whether the function of the skin was recovered and maintained by each of the compositions for skin care prepared in Examples 5 to 7 and Comparative Examples 5 to 7.

In the test method, the inner part of the forearm of a subject was treated with a 5% sodium dodecyl sulfate aqueous solution for 5 minutes, dried at room temperature, and allowed to stand for 1 hour, and then the skin moisture, erythema, and percutaneous moisture loss amount of the test site were measured to define the values as initial measurement values.

Every day, the 5% sodium dodecyl sulfate aqueous solution (hereinafter, SDS aqueous solution) was applied to the inner part of the forearm of the subject once, and then after 2 hours and after 6 hours, each of the compositions for skin care prepared in Examples 5 to 7 and Comparative Examples 5 to 7 was applied thereto twice. The process was repeated every day for 2 weeks, and then the skin moisture, erythema, and percutaneous moisture loss amount of each test site were measured to define the values as final measurement values. The skin improvement effect was evaluated by comparing the final measurement values with the initial measurement values for each test site. The skin measurement on each test site was repeated five times, and the average value was obtained by subjecting 10 subjects whose age ranges from 20 to 40 years old to the test.

As test apparatuses, Mexameter MX18, Corneometer CM825, and Tewameter TM300, which are manufactured by CK Electronic GMBH, Germany, were used, and the results are shown in the following Tables 6 to 8.

**[Table 6]**

| Panel | Example 5 | Example 6 | Example 7 | Comparative Example 5 | Comparative Example 6 | Comparative Example 7 | Control |
|---|---|---|---|---|---|---|---|
| Initial measurement value | 24.3 | 25.3 | 26.2 | 25.2 | 25.8 | 26.0 | 25.2 |
| Final measurement value | 40.4 | 47.4 | 49.3 | 30.3 | 32.3 | 33.3 | 16.4 |
| Increased value in | 16.1 | 22.1 | 23.1 | 5.1 | 6.5 | 7.3 | -8.8 |
| skin moisture | | | | | | | |
| Increase ratio | 66.3% | 87.4% | 88.2% | 20% | 25.2% | 28.1% | -34.9% |

Increase ratio in skin moisture (Control is when only the SDS aqueous solution is applied, and the larger the increase ratio in skin moisture is, the better the effect is)

**[Table 7]**

| Panel | Example 5 | Example 6 | Example 7 | Comparative Example 5 | Comparative Example 6 | Comparative Example 7 | Control |
|---|---|---|---|---|---|---|---|
| Initial measurement value | 225 | 232 | 236 | 229 | 228 | 229 | 230 |
| Final measurement value | 223 | 227 | 228 | 252 | 250 | 251 | 282 |
| Variation value in erythema | -2 | -5 | -8 | 23 | 22 | 22 | 52.3 |
| Increase ratio | -0.9% | -2.16% | -3.39% | 10.04% | 9.65% | 9.61% | 22.74% |

Increase ratio in erythema (Control is when only the SDS aqueous solution is applied, and the smaller the increase ratio in erythema is, the better the effect is)

**[Table 8]**

| Panel | Example 5 | Example 6 | Example 7 | Comparative Example 5 | Comparative Example 6 | Comparative Example 7 | Control |
|---|---|---|---|---|---|---|---|
| Initial measurement value | 15.1 | 15.3 | 15.2 | 15.3 | 15 | 15.1 | 15.2 |
| Final measurement value | 15.9 | 15.4 | 15.3 | 21.2 | 21 | 21.1 | 25.5 |
| Variation value in skin moisture | 0.8 | 0.1 | 0.1 | 5.9 | 6.0 | 6.0 | 10.3 |
| Increase ratio | 5.3% | 0.7% | 0.7% | 38.6% | 40.0% | 39.7% | 67.8% |

Loss ratio in skin moisture (Control is when only the SDS aqueous solution is applied, and the smaller the decrease ratio in skin moisture is, the better the effect is)

As described above, the present invention has been described through the Examples, but is not always limited thereto, and various modified embodiments can be made within the range not departing from the scope and spirit of the present invention. Further, specific situations and materials can be adopted in the teaching of the present invention by making various modifications without departing from the essential range of the present invention. Therefore, the protection scope of the present invention is not limited to the specific embodiment aspect disclosed as the best form planned in carrying out the present invention, and is interpreted to include all the embodiment aspects belonging to the scope of the accompanying claims in the present invention.

### [Industrial Applicability]

The graft copolymer of the present invention exhibits improved moisturizing capacity and excellent biocompatibility, and accordingly, the composition for hair and skin care including the same may also be usefully used for improved moisturizing capacity, protection of damaged hair, skin affinity, and skin barrier function.

## Claims

1. A graft copolymer, comprising:
at least one of phosphorylcholine analogous group-containing first monomers represented by the following Formula (1) in at least one of polysaccharides as a main chain: 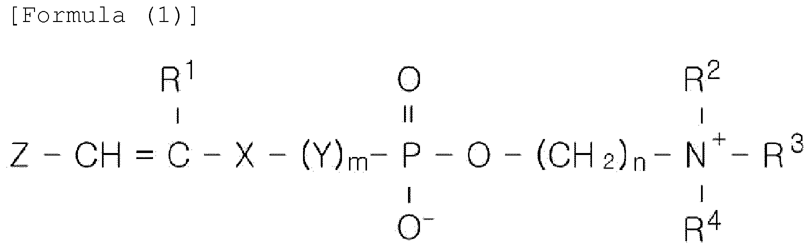
in Formula (1), X represents a divalent organic residue, Y represents an alkyleneoxy group having from 1 to 6 carbon atoms, Z represents a hydrogen atom or R⁵-O-CO-(here, R⁵ represents an alkyl group having from 1 to 10 carbon atoms, or a hydroxyalkyl group having from 1 to 10 carbon atoms), R¹ represents a hydrogen atom or a methyl group, R², R³, and R⁴ are the same or different groups and represent a hydrogen atom and a hydrocarbon group or a hydroxyhydrocarbon group having from 1 to 6 carbon atoms, m is 0 or 1, and n is an integer from 2 to 4.

2. The graft copolymer of claim 1, further comprising:
a second monomer including at least one of quaternary ammonium compounds containing an epoxy group.

3. The graft copolymer of claim 1, wherein the contents of the polysaccharide and the first monomer, which are included in the graft copolymer, are from 30% by weight to 99.5% by weight and from 0.5% by weight to 70% by weight, respectively.

4. The graft copolymer of claim 1, wherein the polysaccharide is hydroxyethyl cellulose, hydroxypropyl cellulose, hydroxyethyl propyl cellulose, guar gum, galactomannon gum, locust bean gum, and starch.

5. The graft copolymer of claim 2, wherein the content of the first monomer in the graft copolymer is from 0.5 part by weight to 50 parts by weight based on 100 parts by weight of the polysaccharide.

6. The graft copolymer of claim 2, wherein the content of the second monomer in the graft copolymer is from 5 parts by weight to 50 parts by weight based on 100 parts by weight of the polysaccharide.

7. The graft copolymer of claim 6, wherein the second monomer is epoxy alkyl trialkyl ammonium halide, the alkyl group of the epoxy alkyl has from 2 to 20 carbon atoms and the alkyl group of the trialkyl has from 1 to 20 carbon atoms, and the alkyl groups have the same or different carbon atoms.

8. The graft copolymer of claim 7, wherein the second monomer is epoxy propyl trimethyl ammonium chloride.

9. The graft copolymer of claim 1, wherein the first monomer is 2-(methacryloyloxy)ethyl-2'-(trimethylammonio)ethylphosphate.

10. The graft copolymer of claim 1, further comprising:
a mixture obtained by mixing one or two or more monomers of diallyl dimethyl ammonium chloride, 2-hydroxyethyl acrylate, stearyl acrylate, butyl acrylate, acrylic acid, methacrylic acid, acrylamide, ethyl acrylate, methyl methacrylate, methyl methacrylate, hydroxyethyl methacrylate, and sodium acryloyl dimethyl taurate.

11. The graft copolymer of claim 1, wherein the graft copolymer has a weight average molecular weight from 1,000 to 2,000,000.

12. A composition for skin and hair care, comprising:
the graft copolymer of any one of claims 1 to 11 as an active ingredient.

13. The composition for skin and hair care of claim 12, wherein the content of the graft copolymer is from 0.01% by weight to 5% by weight based on the total weight of the composition.
